Europäisches Patentamt

(19) European Patent Office

Office européen des brevets

(11) Veröffentlichungsnummer: **0 028 361**

A2

(12) # EUROPÄISCHE PATENTANMELDUNG

(21) Anmeldenummer: 80106459.3

(22) Anmeldetag: 23.10.80

(51) Int. Cl.³: **C 07 D 323/06**
**B 01 J 14/00, B 01 J 19/24**

(30) Priorität: 31.10.79 DE 2943984

(43) Veröffentlichungstag der Anmeldung:
13.05.81 Patentblatt 81/19

(84) Benannte Vertragsstaaten:
AT BE CH DE FR GB IT LI NL SE

(71) Anmelder: HOECHST AKTIENGESELLSCHAFT
Zentrale Patentabteilung Postfach 80 03 20
D-6230 Frankfurt/Main 80(DE)

(72) Erfinder: Bär, Helmut
Wilhelm-Weber-Weg 4
D-6050 Offenbach/Main(DE)

(72) Erfinder: Mader, Herbert
Heinrich-Heine-Strasse 24
D-6085 Nauheim(DE)

(72) Erfinder: Mück, Karl-Friedrich, Dr.
Wittenberger Strasse 17
D-6200 Wiesbaden(DE)

(72) Erfinder: Zorner, Paul
Pfingstbornstrasse 95
D-6200 Wiesbaden(DE)

(54) Verfahren und Vorrichtung zur kontinuierlichen Herstellung von Trioxan.

(57) Die Erfindung betrifft ein Verfahren zur kontinuierlichen Herstellung von Trioxan, gegebenenfalls zusammen mit cyclischen Formalen, aus wäßrigen Formaldehyd-Lösungen in einem Umlaufreaktor mit Verdampfer, wobei das Dampf-/Flüssigkeitsgemisch vor Eintritt in den Reaktor in die Dampf- und in die Flüssigkeitsphase aufgetrennt wird und anschließend beide Phasen getrennt dem Reaktor zugeführt werden. Nach dem erfindungsgemäßen Verfahren lassen sich insbesondere hohe Raumzeitausbeuten erreichen. Weiterhin hat die Erfindung eine Vorrichtung zur Durchführung dieses Verfahrens zum Gegenstand.

./...

HOECHST AKTIENGESELLSCHAFT HOE 79/F 288          Dr.ZR/cr

Verfahren und Vorrichtung zur kontinuierlichen Herstellung von Trioxan

Die Herstellung von Trioxan aus wässriger Formaldehydlösung wird verschiedentlich in der Literatur beschrieben (vgl. Walker, Formaldehyde, Reinhold Publ. New York, 3. Auflage, 1964, Seite 198-199). Das bei höheren Temperaturen in Gegenwart saurer Katalysatoren gebildete Trioxan wird hierbei durch Destillation aus dem Reaktionsgemisch abgetrennt. Der Synthesedampf, der außer Trioxan, Wasser und Formaldehyd noch Nebenprodukte der Synthese enthält, wird meist entsprechend der US-Patentschrift 2 304 080 in einer dem Reaktor aufgesetzten Verstärkerkolonne oder entsprechend der GB-PS 1.012.372 in einer mit Verstärker und Abtriebsteil versehenen Kolonne rektifiziert. Die erhaltene trioxanreiche Fraktion wird durch Extraktion und/oder ein anderes bekanntes Trennverfahren weiter aufgearbeitet.

Es ist bekannt, daß die Zeitausbeuten (g Trioxan pro kg Formaldehyd und Stunde) bei der Trioxansynthese gering sind. Nach der DE-PS 1 135 491 werden z.B. durch einfache Destillation des Reaktionsgemisches aus dem Reaktionsgefäß Zeitausbeuten von 152 g Trioxan pro kg Formaldehyd und Stunde erreicht. Die geringen Zeitausbeuten haben zur Folge, daß bei der Herstellung von Trioxan aus wässrigen Formaldehyd-Lösungen lange Verweilzeiten benötigt werden. Außerdem sind auch große Reaktionsvolumina erforderlich, um technisch zufriedenstellende Raumzeitausbeuten (g Trioxan pro 1 Reaktionsvolumen und Zeit) zu erhalten.

Zur Erhöhung der Raumzeitausbeuten bei der Trioxansynthese wurde bereits vorgeschlagen, das chemische Gleichgewicht zwischen Formaldehyd, Wasser und Trioxan im Reaktionsgemisch möglichst weitgehend durch Arbeiten bei hohen Verdampfungsgeschwindigkeiten zu stören.

Diese Verfahrensweise führt jedoch zu niedrigen Trioxankonzentrationen im Synthesedampf (vgl. E. Bartholomé, Chem.Ing. Techn. 43 (1971), 597). Die Aufarbeitung solcher Reaktionsdämpfe ist wegen der niedrigen Trioxankonzentration sehr energieintensiv.

Weiterhin ist aus der DE-AS 1.543.390 ein Verfahren bekannt geworden, für das als maximaler Wert der Zeitausbeute 1 090 g Trioxan pro kg Formaldehyd und Stunde genannt werden. Nach diesem Verfahren wird wässrige Formaldehyd-Lösung wie üblich in Gegenwart von sauren Katalysatoren in einem Umlaufverdampfer zum Sieden erhitzt, und die trioxanreichen Synthesedämpfe werden dann durch eine, dem Reaktor aufgesetzte Kolonne geleitet. In dieser Kolonne wird dem Synthesedampf ausreagierte Reaktionsflüssigkeit entgegengeführt, die sich im chemischen Gleichgewicht befindet. Hierdurch wird erreicht, daß die Trioxankonzentration der Synthesedämpfe auch bei hohen Verdampfungsgeschwindigkeiten den thermodynamischen Gleichgewichtswert erreicht, der sonst nur bei niedrigen Verdampfungsgeschwindigkeiten und totaler Durchmischung erhalten wird und der dem Phasengleichgewicht von Trioxan in gasförmiger und flüssiger Phase entspricht.

Nachteil des Verfahrens gemäß der DE-AS 1.543.390 ist jedoch, daß Zusatzaggregate benötigt werden, die eine erhebliche Vergrößerung des eigentlichen Reaktorvolumens und damit eine Erniedrigung der Raumzeitausbeuten bedeuten. So besteht in dem beschriebenen Verfahren der mit der Reaktionsflüssigkeit in Kontakt kommende Verfahrensteil aus dem eigentlichen Reaktor, einem Verdampfer, einer Pumpe, einer langen Rohrleitung, gegebenenfalls mit Verweilgefäß und einer Kolonne mit Einbauten.

All diese Teile müssen aus Materialien gefertigt sein, die gegenüber einer ca.100-110°C heißen sauren, z.B. schwefelsauren Formaldehyd-Lösung beständig sind.Die generellen Nachteile von korrosionsbeständigen Reaktoren sind in der DE-AS 2.103.687 beschrieben. Berechnet man nun die Raumzeitausbeuten, dann ergeben sich wesentlich geringere Werte als für die in der DE-AS 1.543.390 angegebenen Zeitausbeuten.

In der DE-AS 2.428.719 wird ein Verfahren zur Abtrennung von Trioxan aus wässrigen Lösungen, die Trioxan zusammen mit Formaldehyd enthalten, beschrieben, wonach 5 bis 15 Gew.-% der Lösungen bei Temperaturen unter 100°C unter vermindertem Druck und bei Verweilzeiten von weniger als 1 Minute abdestilliert werden und anschließend das Trioxan aus dem Destillat isoliert wird. Nachteilig an dieser Verfahrensweise sind die geringen Zeitausbeuten.

Schließlich ist auch bereits vorgeschlagen worden, hohe Raumzeitausbeuten an Trioxan auch bei hohen Durchsätzen dadurch zu erreichen, daß in einem Zwangsumlaufreaktor das den Verdampfer verlassende Dampf-Flüssigkeitsgemisch zur Erreichung des Phasengleichgewichts unter den Flüssigkeitsspiegel der ausreagierten Reaktionsmischung im Reaktor eingeleitet wird und der Synthesedampfstrom aus dem Kopf des Reaktors austritt.

Die vorliegende Erfindung betrifft nun ein Verfahren zur kontinuierlichen Herstellung von Trioxan aus wässrigen Formaldehyd-Lösungen in Gegenwart saurer Katalysatoren in einem Zwangsumlaufreaktor mit Verdampfer bei Verweilzeiten zwischen 2 und 240 Minuten, wobei das den Verdampfer verlassende Dampf-Flüssigkeitsgemisch unter den Flüssigkeitsspiegel der Reaktionsmischung im Reaktor eingeleitet wird und der Synthesedampf aus dem Reaktor, vorzugsweise aus seinem Kopfbereich austritt, dadurch gekennzeichnet, daß das Dampf-Flüssigkeitsgemisch vor Eintritt in den Reaktor in die Dampf- und in die Flüssigkeitsphase aufgetrennt wird und anschließend beide Phasen getrennt in den Reaktor zurückgeführt werden.

Weiterhin hat die Erfindung ein kontinuierliches Verfahren zur gleichzeitigen Herstellung von Trioxan und cyclischen Formalen zum Gegenstand, wobei eine wässrige Formaldehyd-Lösung, die mindestens ein Diol und/oder mindestens ein Epoxyd enthält, in der vorstehenden Weise behandelt wird.

Schließlich betrifft die Erfindung noch die Vorrichtung gemäß den Ansprüchen 4 bis 7.

Nach dem erfindungsgemäßen Verfahren werden in überraschender Weise Trioxankonzentrationen im Synthesedampf auch bei hohen Verdampfungsgeschwindigkeiten erhalten, die dem Gleichgewichtswert nahezu entsprechen; gleichzeitig werden Pulsationserscheinungen, wie sie bei ungleichmäßiger Verdampfung und bei Zweiphasenströmung zu erwarten sind, weitgehend vermieden.

Die Trimerisierungsreaktion von Formaldehyd zu Trioxan erfolgt in an sich bekannter Weise durch Umsatz von wässrigen, im allgemeinen 30-80 %igen, vorzugsweise 40-70%igen Formaldehyd-Lösungen, gegebenenfalls unter Zusatz der bekannten Antischaummittel, in Gegenwart der hierfür bekannten sauren Katalysatoren, wie Mineralsäuren, starken organischen Säuren oder einer, in seiner katalytischen Aktivität entsprechenden Menge eines anderen sauren Katalysators. Als saure Katalysatoren, die weniger flüchtig als das Reaktionsgemisch sein müssen, haben sich beispielsweise Schwefelsäure, Phosphorsäure, p-Toluolsulfonsäure oder saure Ionenaustauscher als brauchbar erwiesen. Die Menge ist nicht kritisch und beträgt in der Regel 2 bis 25 %, vorzugsweise 2 bis 10 %.

Falls nach der erfindungsgemäßen Variante ein Gemisch aus Trioxan und mindestens einem cyclischen Formal hergestellt werden soll, werden der wässrigen Formaldehyd-Lösung zweckmäßigerweise 1 bis 25 Gew.-%, vorzugsweise 2 bis 15 Gew.-%, bezogen auf Formaldehyd, an mindestens einem Diol und/oder mindestens einem Epoxyd zugesetzt.

Die hierfür in Frage kommenden Diole sind vor allem 1,2-Diole, 1,3-Diole und $\alpha,\omega$-Diole. Ebenso können statt der 1,2-Diole auch die entsprechenden Epoxyde oder Gemische aus beiden eingesetzt werden. Vorzugsweise werden Diole verwendet, deren cyclische Formale Siedepunkte von kleiner als 150°C haben und/oder mit Wasser niedrigsiedende Azeotrope (< 150°C) bilden oder wasserdampfflüchtig sind. Als geeignet haben sich z.B. Äthylenglykol, Äthylenoxyd, Propylenglykol-1,2, Propylenoxyd, Propylenglykol-1,3, Butandiol-1,2, Butandiol-1,3, Butandiol-1,4 und Buten(3)-diol-1,2 erwiesen. Vorzugsweise werden erfindungsgemäß dabei Äthylenglykol bzw. Äthylenoxyd, Propylenglykol-1,2 und Butandiol-1,4 eingesetzt und besonders bevorzugt Äthylenglykol bzw. Äthylenoxyd.

Die Verweilzeit der Reaktionsmischung im Reaktorsystem beträgt 2 bis 240 Minuten, vorzugsweise 5 bis 120 Minuten und besonders bevorzugt 15 bis 60 Minuten. Die Temperaturen der Reaktionsmischung liegen je nach Druck zwischen 50°C und 150°C, vorzugsweise 95°C und 110°C.

Das Reaktionsprodukt bestehend aus Trioxan, Formaldehyd und Wasser sowie ggf. cyclischen Formalen wird im Umlaufverdampfer verdampft. Hierzu kann bei Normaldruck, unter vermindertem Druck, beispielsweise zwischen 150 und 950 mbar oder unter Überdruck, beispielsweise 1 - 4 bar, gearbeitet werden. Vorzugsweise wird bei Normaldruck bzw. leichtem Überdruck gearbeitet.

Der Verdampfungsgrad (Quotient aus verdampfter Produktmenge und durch den Verdampfer umlaufende Produktmenge x 100) der Reaktionsmischung im Verdampfer liegt beim erfindungsgemäßen Verfahren, beispielsweise zwischen 0,1 bis 25 %, vorzugsweise zwischen 1 und 20 % und besonders vorteilhaft zwischen 6 und 14 %.

Entsprechend dem erfindungsgemäßen Verfahren wird das den Verdampfer verlassende Dampf-Flüssigkeits-Gemisch mittels hierfür bekannter Vorrichtungen, wie Abscheider, in die Flüssigkeitsphase und in die Dampfphase aufgetrennt und durch getrennte Rohrleitungen dem Reaktorbehälter, die vorzugsweise oberhalb des Flüssigkeitsspiegels in diesen einmünden, zugeführt. Zumindestens die Rohrleitung für den Dampf, die vorzugsweise im Bereich des Reaktorkopfes, speziell in der Nähe von dessen höchstem Punkt oder direkt an dessen höchstem Punkt, in den Reaktor eintritt, ist dabei als Tauchrohr ausgebildet, d.h. es reicht bis unterhalb des Flüssigkeitsspiegels. Das Abtauchen in die Reaktionsmischung sollte dabei so tief wie möglich vorgenommen werden, damit ein optimaler Stoffaustausch und eine optimale Vermischung und Begasung des Reaktorinhalts (Mammutpumpenprinzip) gewährleistet ist. Die Eintauchtiefe sollte beispielsweise 20-80 %, vorzugsweise 30-70 % des verwendeten Reaktorfüllstandes betragen. Dabei müssen Flüssigkeitsstand im Reaktorbehälter, Abtauchtiefe, Saugleitung, Pumpenart und Pumpenanordnung so aufeinander abgestimmt sein, daß keine Kavitation im Pumpenlaufrad auftreten kann, da sich die Reaktionsflüssigkeit in siedendem Zustand befindet.

Zur Verbesserung des Stoffaustausches zwischen Reaktorflüssigkeit und der aus dem Verdampfer kommenden gasförmigen Phase kann eine zusätzliche innige Durchmischung und Begasung des Reaktorinhalts durch hierfür bekannte Verteiler (Trennelemente), wie zylindrisch um das Tauchrohr angeordnete Strömungsbleche erfolgen.

Auch die den Abscheider verlassende Flüssigphase wird vorzugsweise mit einer ausreichenden Eintauchtiefe unterhalb des Flüssigkeitsspiegels in die Reaktorflüssigkeit eingeleitet, um ein Durchschlagen des dampfförmigen Anteils durch dieses Rücklaufrohr zu verhindern.

Das das Reaktorsystem verlassende Synthesedampf-Gemisch wird in üblicher Weise entweder als Dampf oder als Kondensat einer Rektifikation zugeleitet, wie z.B. in der GB-PS 1.012.372 beschrieben, und dabei destillativ angereichert. Die anfallende trioxanreiche Fraktion, die gegebenenfalls auch cyclische Formale enthält, kann dann z.B. durch Extraktion mit einem mit Wasser nichtmischbaren Lösungsmittel für Trioxan und ggf. für cyclische Formale, wie Methylenchlorid und anschließender Neutralisation und fraktionierte Destillation oder Kristallisation gereinigt werden. Auch andere bekannte Trennverfahren können hierfür Einsatz finden, wie beispielsweise in Process Exonomics Program, Stanford Institut Report 23 (1967) S. 181 oder in der DE-OS 1.570.335 beschrieben.

Das erfindungsgemäße Verfahren ist verfahrenstechnisch besonders vorteilhaft, da hierbei außer z.B. einer zwischen Reaktor und Verdampfer installierten Pumpe keine Vergrößerung des Reaktionsraums durch Zusatzaggregate notwendig ist, um auch bei hohen Verdampfungsgeschwindigkeiten Synthesedampf mit dem höchstmöglichen Trioxangehalt, der dem Gleichgewichtswert in der Gasphase für das Verteilungsgleichgewicht von Trioxan, Wasser und Formaldehyd in flüssiger und gasförmiger Phase entspricht, zu erzeugen. Nach früher beschriebenen Verfahren ist dieser Trioxangehalt nur bei Arbeiten mit niederen Verdampfungsgeschwindigkeiten oder nach der in der DE-AS 1.543.390 beschriebenen, aufwendigeren Verfahrensweise realisierbar. Erfindungsgemäß sind somit Raumzeitausbeuten erreichbar, die wesentlich höher sind als die nach den früher beschriebenen Verfahren erzielten.

Das erfindungsgemäße Verfahren ermöglicht außerdem eine Trioxansynthese mit einem minimalen spezifischen Energiebedarf; es weist also eine günstige Energiebilanz auf.

Weiterhin zeichnet sich die Verfahrensweise gemäß der Erfindung dadurch aus, daß durch die kurzen Verweilzeiten bei Anwendung hoher Verdampfungsgeschwindigkeiten, die Bildung von Nebenprodukten wie z.B. Ameisensäure zurückgedrängt wird. In gleicher Weise wirken sich geringe Katalysatorsäure-Konzentrationen oder die Verwendung von Ionenaustauscher aus.

Die Reaktion wird in einem bekannten Zwangsumlaufreaktor mit Verdampfer durchgeführt, wobei erfindungsgemäß dem Verdampfer eine Trennvorrichtung (Abscheider) für die Dampf- und Flüssigphase nachgeschaltet ist. Besonders geeignet sind Zwangsumlaufverdampfer, bestehend aus Reaktionskessel, Pumpe und Verdampfer, wie sie zum Beispiel in Ullmann, Bd. 1 (1951), 3. Auflage, Seiten 533-537 beschrieben sind.

Der Reaktor ist dabei vorzugsweise in stehender zylindrischer Bauweise ausgebildet und weist eine Nachdosierleitung für die Ausgangsmischung und eine Leitung zum Abzug des Destillates auf. Die Nachdosierleitung befindet sich dabei vorzugsweise am unteren Ende des Reaktors, während die Abzugsleitung vorzugsweise am Reaktorkopf angebracht ist.

Vorzugsweise am unteren Ende des Reaktors befindet sich auch die Rohrleitung, die über eine Pumpe zum Verdampfer führt. Die Fördermenge der Pumpe ist dabei nicht kritisch und kann innerhalb weiter Bereiche, beispielsweise von 30 bis 300 $m^3$/h schwanken. Die den Verdampfer verlassende Rohrleitung mündet dann in die Abscheidevorrichtung üblicher Bauart zur Trennung der Dampf- und Flüssigphase. Von dieser Abscheidevorrichtung gehen dann zwei Rohrleitungen zur Aufnahme der Dampf- bzw. Flüssigphase aus, die in den Reaktor eintreten, wobei zumindestens das Dampfleitungsrohr als Tauchrohr mit Begasungseinrichtung ausgebildet ist. Um dieses Tauchrohr befinden sich vorzugsweise Verteiler(Trennelemente), wie zylindrisch angeordnete Strömungsbleche, die eine Zirkulationsströmung in der Begasungszone nach oben und eine Rückströmung der abgekühlten Flüssigkeit zum Behälterboden hin begünstigen.

Die Rohrleitung für den Dampf tritt vorzugsweise im Bereich des Reaktorkopfes in den Reaktor ein, um auf diese Weise asymetrische Reaktionskräfte , die bei seitlichem Eintritt auftreten und zu Schädigungen des an der Eintrittsstelle vorhandenen Stutzens und der umgebenden Reaktorinnenwand führen können, weitgehend zu vermeiden. Dies wird dann am besten erreicht, wenn diese Rohrleitung an der höchsten Stelle des Reaktorkopfes einmündet und dementsprechend im Reaktor in Richtung der Reaktorlängsachse verläuft.

Auch das Leitungsrohr für die Flüssigphase, das in der Regel im zylindrischen Teil seitlich in den Reaktor einmündet, reicht erfindungsgemäß vorzugsweise unter den Flüssigkeitsspiegel, ist also gleichfalls als Tauchrohr ausgebildet. Dabei ist die Eintauchtiefe so ausreichend zu wählen, daß ein Durchschlagen des dampfförmigen Anteils durch das Rücklaufrohr vermieden wird. Dies ist dann der Fall, wenn die Eintauchtiefe zumindestens gleich groß wie die des Tauchrohres für die Dampfphase ist. Vorzugsweise ist sie jedoch im ersten Fall um mindestens 25 % größer als beim Tauchrohr für die Dampfphase.

Für den Fall, daß die Rohrleitung für die Flüssigphase nicht als Tauchrohr ausgebildet ist, was erfindungsgemäß auch möglich ist, muß durch geeignete Maßnahmen dafür Sorge getragen werden, daß kein Dampfdurchschlag erfolgen kann. Beispielsweise kann dies in der Weise erfolgen, daß die den Abscheider verlassende Flüssigkeitsrohrleitung vor Eintritt in den Reaktor als U-Rohr mit einer Schenkellänge von mindestens der Eintauchtiefe des Tauchrohres für die Dampfphase ausgebildet ist (Siphon).

Unter den Rahmen der Erfindung fällt auch die Variante, wonach die Flüssigkeitsrohrleitung unterhalb der Flüssigkeitsoberfläche in den Reaktor einmündet.

Zur Reinigung des Reaktors ist zweckdienlich ein Mannloch im oberen Teil vorgesehen. Außerdem ist der Reaktor zweckmäßigerweise gegen Wärmeabstrahlung isoliert. Er kann auch Mittel zum Temperieren der Reaktorwand enthalten, beispielsweise einen von einem Temperiermedium durchflossenen Doppelmantel sowie Rohr- oder Halbrohrschlangen oder elektrische Heizelemente.

Daneben kann der Reaktor auch die üblichen Einbauten und Meßvorrichtungen, beispielsweise für Temperatur und Standhöhe der Flüssigkeit enthalten. Evtl. mitgerissene Flüssigkeitsteilchen im den Reaktor verlassenden Synthesedampf können durch einen am Beginn der Gasabzugsleitung befindlichen sog. Demister aus korrosionsbeständigem Werkstoff zurückgehalten werden.

Die anliegende Figur zeigt ein Beispiel für eine erfindungsgemäße Vorrichtung. Der zylindrische, aufrechtstehende Reaktorbehälter (1) weist eine Nachdosierleitung (2) und die Gasabzugsleitung (3) auf. In der zum Verdampfer (6), hier speziell ein Röhrenbündelwärmeaustauscher, führenden Rohrleitung (4) ist eine Pumpe (5) zwischengeschaltet. Die den Verdampfer verlassende Leitung (7) mündet in den Abscheider (8), von dem das Dampfleitungsrohr (9) und das Flüssigkeitsrohr (10) ausgehen. Beide sind als Tauchrohre ausgebildet, d.h. sie laufen bis unter den Flüssigkeitsspiegel (11). Das Tauchrohr (9) endet mit einer Begasungseinrichtung (9a). Es ist von einem zylindrischen Verteiler (Trennelement) (12) konzentrisch umgeben zum Zwecke des besseren Stoffaustausches zwischen flüssiger und gasförmiger Phase.

Die folgenden Beispiele sollen die Erfindung näher erläutern.

Beispiele

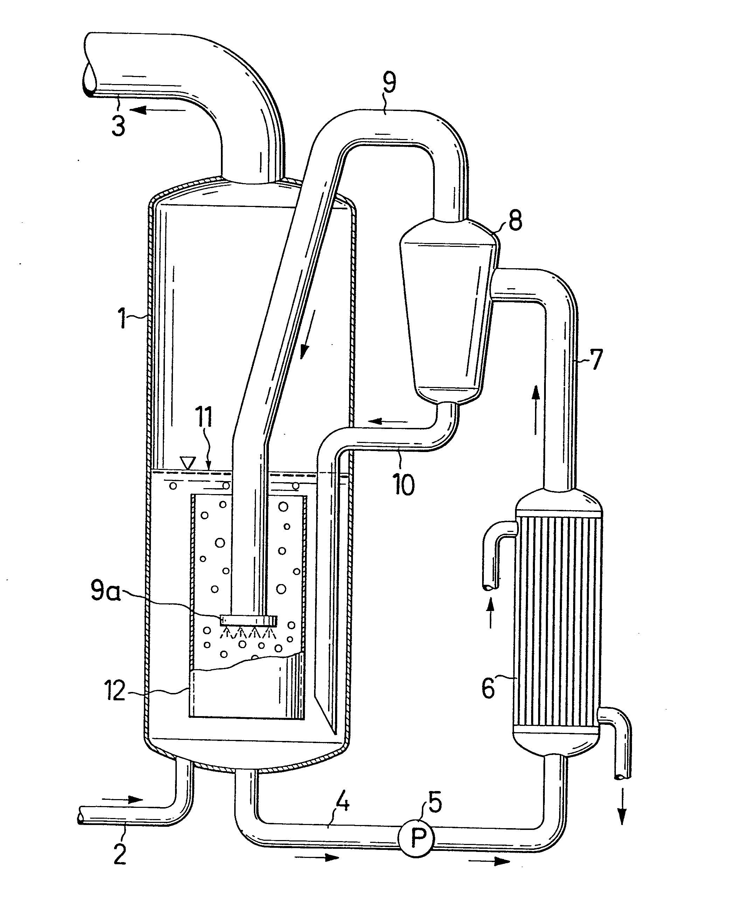

Die Reaktion wurde in der Vorrichtung gemäß Figur 1 durchgeführt; die Eintauchtiefe des Dampfleitungsrohres betrug 70 % des Reaktorfüllstandes.

Im Reaktionskessel wurden jeweils 1 000 g einer Mischung, bestehend aus 90 Teilen einer 63,5%igen wässrigen Formaldehydlösung und 10 Teilen konzentrierter Schwefelsäure vorgelegt. Der Kessel war auf 95°C temperiert. Das Gemisch wurde mittels der Pumpe durch den Verdampfer gepumpt, wobei dieser je nach der gewünschten Synthesedampfmenge (Trioxan-Durchsatz) verschieden stark beheizt wurde. Der das System verlassende Synthesedampf wurde in einem Quenchsystem total kondensiert und das Kondensat auf Trioxan und Formaldehydgehalt untersucht. Entsprechend dem verdampften Anteil wurde 63,5%ige Formaldehydlösung nachdosiert. Die Versuchsdauer betrug jeweils 6 Stunden.

Die Ergebnisse sind zusammen mit den Vergleichsversuchen in Tabelle 1 und Tabelle 2 zusammengestellt.

Man sieht aus Tabelle 1 (Beispiele 1 - 3), daß die Trioxankonzentrationen im Synthesedampf unabhängig vom Verdampfungsgrad im Verdampfer sind.

Tabelle 1

| Beispiel Nr. | Trioxan im Destillat | Verdampfungsgrad | Umsatz | Verweilzeit |
|---|---|---|---|---|
| 1 | 22,4 | 5,4 | 35,3 | 0,8 |
| 2 | 21,3 | 1,16 | 33,5 | 0,94 |
| 3 | 21,6 | 4,35 | 34,0 | 0,92 |

Aus Tabelle 2 kann man erkennen, daß bei identischen Verweilzeiten die Trioxankonzentrationen im Synthesedampf sowohl beim erfindungsgemäßen Verfahren als auch beim Vergleichsbeispiel (gemäß DE-AS 1.543.390) größer als 20 Gewichtsprozent sind.

Das erfindungsgemäße Verfahren zeichnet sich aber durch wesentlich höhere Raumzeitausbeuten, bedingt durch die bedeutend kleineren Reaktionsvolumina als beim Vergleichsverfahren (DE-AS 1.543.390) aus.

Tabelle 2

| Beispiel Nr. | Formaldehyd-konzentration Einlauf % | Verweilzeit h | Trioxan im Destillat % | Reaktorvolumen | Verhältnis der Reaktions-volumina | Umsatz zu Trioxan % | Verhältnis der Raumzeit-Ausbeuten |
|---|---|---|---|---|---|---|---|
| 4 | 63,5 | 0,3 | 22,0 | Reaktor, Verdampfer, Pumpe, | 1 | 34,6 | 1 |
| 5 * | 63,5 | 0,3 | 20,8 | Reaktor, Pumpe, Verdampfer, Kolonne, lange Rohrleitung | 1,5 - 2 | 32,8 | 0,68 - 0,51 |

* gemäß Beispiel 2 der DE-AS 1.543.390

- 14 -

PATENTANSPRÜCHE

1. Verfahren zur kontinuierlichen Herstellung von Trioxan, gegebenenfalls zusammen mit cyclischen Formalen, aus wässrigen Formaldehyd-Lösungen, die gegebenenfalls mindestens ein Diol und/oder mindestens ein Epoxyd enthalten, in Gegenwart von sauren Katalysatoren in einem Zwangsumlaufreaktor mit Verdampfer bei Verweilzeiten von 2 bis 240 Minuten, wobei das den Verdampfer verlassende Dampf-Flüssigkeitsgemisch unter den Flüssigkeitsspiegel der Reaktionsmischung im Reaktor eingeleitet wird und der Synthesedampf aus dem Reaktor austritt, dadurch gekennzeichnet, daß das Dampf-Flüssigkeitsgemisch vor Eintritt in den Reaktor in die Dampf- und in die Flüssigkeitsphase aufgetrennt wird und anschließend beide Phasen getrennt in den Reaktor zurückgeführt werden.

2. Verfahren nach Anspruch 1, dadurch gekennzeichnet, daß das den Verdampfer verlassende Dampf-Flüssigkeitsgemisch 30 bis 70 % unterhalb des Flüssigkeitsspiegels eingeleitet wird.

3. Verfahren nach Anspruch 1 und 2, dadurch gekennzeichnet, daß die das System verlassende Dampfmenge, bezogen auf die durch den Verdampfer umlaufende Produktmenge, 6 bis 14 % beträgt.

4. Vorrichtung zur kontinuierlichen Herstellung von Trioxan, bestehend aus dem Reaktor mit einer unterhalb des Flüssigkeitsspiegels einmündenden Nachdosierleitung für die Ausgangsmischung und einer oberhalb des Flüssigkeitsspiegels befindlichen Gasabführung, einer unterhalb des Flüssigkeitsspiegels austretenden, über eine Pumpe zum Verdampfer führenden Rohrleitung sowie einer Verbindung zwischen Verdampfer und Reaktor zur Ermöglichung eines Umlaufes, dadurch gekennzeichnet, daß dem Verdampfer eine Abscheidevorrichtung zur Trennung von Dampf- und Flüssigphase nachgeschaltet ist und von dieser Abscheidevorrichtung zwei Rohrleitungen zur Aufnahme der Dampf- bzw. Flüssigphase in den Reaktor führen, wobei zumindestens die Rohrleitung für den Dampf bis unterhalb des Flüssigkeitsspiegels im Reaktor reicht.

5. Vorrichtung nach Anspruch 4, dadurch gekennzeichnet, daß auch die Rohrleitung für die Flüssigphase als Tauchrohr mit einer zur Vermeidung eines Dampfdurchschlages ausreichenden Tauchtiefe ausgebildet ist.

6. Vorrichtung nach Anspruch 4 und 5, dadurch gekennzeichnet, daß die den Dampf aufnehmende Rohrleitung im Bereich des Reaktorkopfes in den Reaktor einmündet.

7. Vorrichtung nach Anspruch 4 bis 6, dadurch gekennzeichnet, daß im Bereich der Austrittsöffnung der Dampfrohrleitung eine Begasungsvorrichtung sowie konzentrische Verteiler (Trennelemente) angebracht sind.